Europäisches Patentamt

European Patent Office

Office européen des brevets

(19)

(11) Veröffentlichungsnummer: **0 376 099**

**A2**

## (12) EUROPÄISCHE PATENTANMELDUNG

(21) Anmeldenummer: 89123315.7

(22) Anmeldetag: 16.12.89

(51) Int. Cl.5 **A62B 18/00**

(30) Priorität: 23.12.88 DE 3843486

(43) Veröffentlichungstag der Anmeldung:
**04.07.90 Patentblatt 90/27**

(84) Benannte Vertragsstaaten:
**DE FR GB IT**

(71) Anmelder: **Drägerwerk Aktiengesellschaft**
**Moislinger Allee 53-55**
**D-2400 Lübeck 1(DE)**

(72) Erfinder: **Van der Smissen, Carl-Ernst, Dr.**
**Am Traveeck 30**
**D-2400 Lübeck(DE)**
Erfinder: **Walther, Hans-Joachim**
**Karpfenstrasse 12**
**D-2400 Lübeck(DE)**
Erfinder: **Eckstein, Wolfgang**
**Friedrich-Ebert-Ring 19**
**D-2407 Sereetz(DE)**

(54) **Atemschutzgerät mit Gebläseunterstützung und Regeneration der Atemfilter.**

(57) Ein Atemschutzgerät mit Gebläseunterstützung für die Durchströmung von mindestens zwei Filtern in der Atemgasführung soll derart verbessert werden, daß die Durchströmung der Filter auch bei deren kleinem Volumen nicht zu einem vorzeitigen Durchbruch bzw. ihrer Erschöpfung führt. Der Tragekomfort soll erhalten bleiben und die Gebläseunterstützung in ihrer Wirkung nicht verringert werden. Dazu ist vorgesehen, daß jeweils eines der Filter zeitweise in Einatemrichtung und das andere zeitweise in Ausatemrichtung durchströmbar, und ihre Durchströmungsrichtung periodisch umkehrbar ist.

Fig.1

EP 0 376 099 A2

## Atemschutzgerät mit Gebläseunterstützung und Regeneration der Atemfilter

Die Erfindung betrifft ein Atemschutzgerät mit Gebläseunterstützung für die Durchströmung von mindestens zwei Filtern in der Atemgasführung.

Atemschutzgeräte mit Filtern, die durch den Geräteträger selbst beatmet werden, zeigen während des Gebrauches unerwünscht hohe Atemwiderstände durch die Filterfüllungen hindurch. Es ist bekannt (GB-A 2 058 577, DE-GM 79 14 929), den erhöhten Atemwiderstand durch Hilfsgebläse zu überwinden und die Atmung zu unterstützen. Damit lassen sich die Atemwiderstände für den Geräteträger bis auf unmerkliche Werte absenken. Da bei gebläseunterstützter Atmung jedoch erheblich größere Luftmengen durch das Filter geleitet werden, um mögliche Beatmungsspitzen bei angestrengter Tätigkeit des Geräteträgers abdecken zu können, werden diese hohen Luftmengen auch dann durch die Filter befördert, wenn sie zur Atmungsunterstützung nicht notwendig sind. Somit werden die Filter unnötig belastet, was zu einem frühzeitigen Filterdurchbruch führt und damit die Einsatzdauer der Atemschutzgeräte beschränkt. Um die Standzeit der Filter zu erhöhen und die Einsatzdauer der Geräte zu verlängern, müßten die Filter voluminös mit genügend Filtermaterial ausgestaltet werden. Das würde zu unhandlichen Geräten mit geringem Tragekomfort durch das hohe Gewicht und das sperrige Volumen des Gerätes führen.

Der vorliegenden Erfindung liegt die Aufgabe zugrunde, ein Atemschutzgerät der genannten Art so zu verbessern, daß die Durchströmung der Filter auch bei deren kleinem Volumen nicht zu einem vorzeitigen Durchbruch bzw. einer Erschöpfung des Filters führt. Der Tragekomfort soll erhalten bleiben und die Gebläseunterstützung in ihrer Wirkung nicht verringert werden.

Zur Lösung dieser Aufgabe ist vorgesehen, daß jeweils eines der Filter zeitweise in Einatemrichtung und das andere zeitweise in Ausatemrichtung durchströmbar, und ihre Durchströmungsrichtung periodisch umkehrbar ist.

Der Vorteil der Erfindung liegt im wesentlichen darin, daß nunmehr durch Ausnutzung der Desorption der von den Filtern aufgenommenen Gase während ihrer Durchströmung in Ausatemrichtung die Filterbelastung zumindest teilweise rückgängig gemacht wird, so daß eine gewisse Regeneration des Filtermaterials verwirklicht wird. Hierzu wird das in Einatemrichtung durchströmte Filter nur während einer kurzen Zeitperiode von der verunreinigten Außenluft durchströmt. Nach Ablauf der vorgebbaren Zeitperiode wird die Durchströmungsrichtung durch das Filter umgekehrt, so daß nunmehr das beladende Filter durch die Ausatemluft gereinigt wird, wobei ein großer Teil der aufgenommenen Schadstoffe desorbiert und aus dem Filter ausgetragen wird. Während der Desorptionszeit des einen Filters, welches nun in Ausatemrichtung durchströmt wird, erfolgt die Einatmung durch das andere Filter, welches nunmehr in Einatemrichtung durchströmt wird und die Schadstoffe aus der Außenluft aufnimmt, bevor sie zum Atemanschluß des Geräteträgers geführt wird. Nach Abschluß der Regenerationszeit des ersten Filters erfolgt eine erneute Umschaltung der Durchströmungsrichtung, so daß das regenerierte Filter wiederum in Einatemrichtung durchströmt wird und das vorher benutzte Filter nunmehr in Ausatemrichtung durchströmt und somit regeneriert wird. Durch diesen wechselweisen Betrieb der beiden Filter jeweils in Einatem- und Ausatemrichtung wird ein periodisches Sammeln des Schadstoffes und Reinigen der Filter durch Ausblasen in die Umgebungsluft erzielt.

Mit der Erfindung werden insbesondere niedrig siedende Gase, welche eine geringe Adsorptionsfähigkeit auf dem Adsorbens des Filters aufweisen und leicht wieder desorbiert werden können, wirksam von dem Geräteträger ferngehalten, ohne daß hierfür ein großvolumiges Filter nötig wäre. Durch die periodische Desorption dieser leichtflüchtigen Stoffe können derartige Filter mehrmals weitgehend regeneriert werden. Ohne die erfindungsgemäße Umkehrung der Durchströmungsrichtung müßten nämlich für solche Stoffe besonders hohe Filterkapazitäten vorgesehen werden.

Eine besonders bevorzugte Ausführungsform der Erfindung besteht darin, daß sowohl im Einatemzweig als auch im Ausatemzweig der Atemgasführung ein Gebläse angeordnet ist. Die Förderleistung der Gebläse kann unabhängig voneinander so abgestimmt werden, daß unter dem Atemanschluß drucklos ein- und ausgeatmet werden kann, wodurch das Atmen besonders angenehm wird.

Um die kurze Förderunterbrechung während der Umschaltzeit eines umschaltbaren Gebläses zu vermeiden, ist vorgesehen, die Umkehr der Durchströmungsrichtung durch ein Umschaltventil im Atemkreis zu verwirklichen. Bei dieser Ausführungsform kann das Gebläse mit seiner Förderleistung kontinuierlich auch während des Umschaltvorgangs arbeiten, so daß nach der kurzen Umschaltzeit das gewünschte Fördervolumen sofort wieder zur Verfügung steht.

Bei einer weiteren vorteilhaften Ausgestaltung der Erfindung werden zwischen den Filtern und dem Atemanschluß Trockenpatronen geschaltet, um zu verhindern, daß die Filter durch hohe Ausatemfeuchtigkeit ungebührlich belastet werden.

Ein Ausführungsbeispiel der Erfindung wird anhand der Zeichnung dargestellt und im folgenden näher erläutert.

Es zeigen:

Fig. 1 die schematische Darstellung des Atemschutzgerätes mit zwei Gebläsen,

Fig. 2 ein Umschaltventil mit dazugehörigem Gebläse.

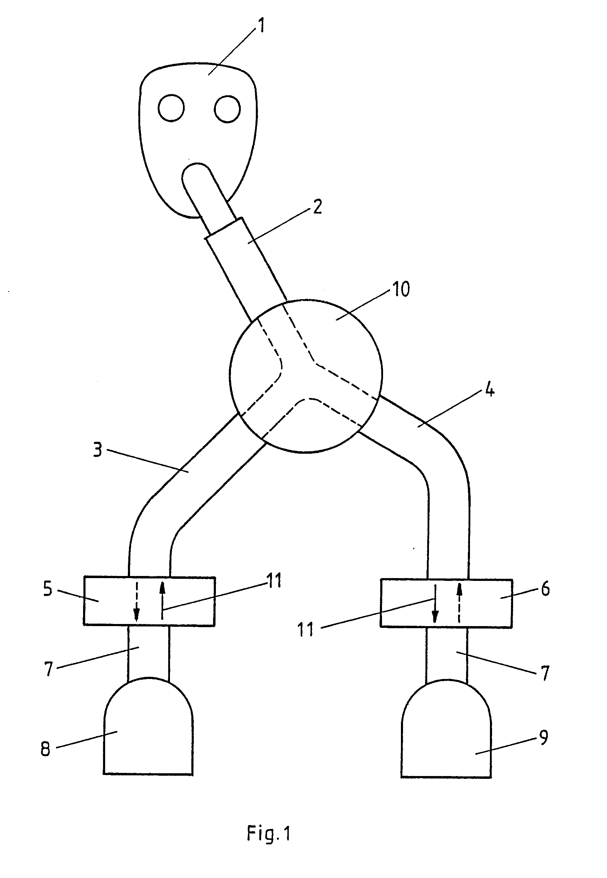

In Fig. 1 ist ein Atemschutzgerät schematisch in seinen wesentlichen Bestandteilen dargestellt. Eine als Atemanschluß dienende Schutzmaske (1) ist über eine Anschlußleitung (2) an einen Wegeverteiler (10) angeschlossen, von dem aus sowohl ein Einatemzweig (3) als auch ein Ausatemzweig (4) ausgehen. Zur Umgebung hin setzen sich der Einatemzweig (3) und der Ausatemzweig (4) in einem Einlaßgebläse (5) und einem Auslaßgebläse (6) fort, die jeweils über eine Trockenpatrone (7) an ein Einlaßfilter (8) und Auslaßfilter (9) angeschlossen sind.

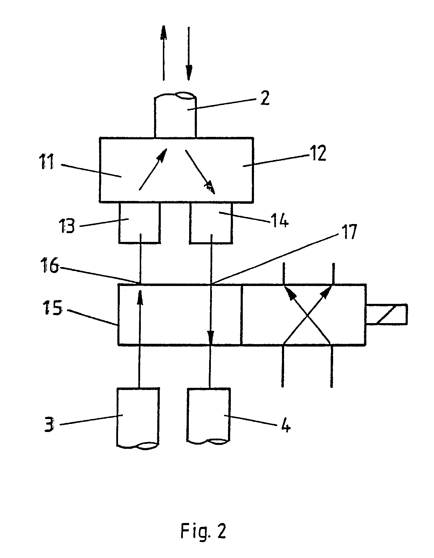

In Fig. 2 ist ein Umwälzgebläse (12) dargestellt, an dessen Strömungsanschlüssen (13, 14) ein Umschaltventil (15) angeschlossen ist. Umwälzgebläse (12) und Umschaltventil (15) können gemeinsam an die Stelle des Wegeverteilers (10) eingesetzt werden und dessen Funktion mit übernehmen, wobei die einzelnen Einlaßgebläse (5) und Auslaßgebläse (6) wegfallen können. Das Umschaltventil (15) ist entweder von Hand oder durch einen nicht dargestellten elektromagnetischen Antrieb betätigbar. Die Ausgänge (16, 17) des Ventils (15) sind mit dem Umwälzgebläse (12) derart verbunden, daß in der dargestellten Grundstellung des Ventils (15) der Einlaßanschluß (13) des Gebläses (12) mit dem Einatemzweig (3) und der Auslaßanschluß (14) mit dem Ausatemzweig (4) verbunden ist. In der Schaltstellung wird die Verbindung zwischen den Anschlüssen (13, 14) und den Zweigen (3, 4) überkreuzt.

Die in der Figur 1 dargestellte Bezeichnung von Einatemzweig (3) und Ausatemzweig (4) gilt für die Betriebsart, in welcher die Gebläse (5, 6) so beschaltet sind, daß ihre Förderrichtung eine Durchströmung der Filter (8, 9) in Richtung der ausgezogenen Richtungspfeile (11) bewirken. Dabei wird das Einlaßfilter (8) von der Umgebungsluft durchströmt und hält die vorhandenen Schadstoffe zurück, so daß gereinigte Umgebungsluft zum Geräteträger über den Atemanschluß (1) geliefert wird. Die Ausatemluft sowie die überschüssige Menge an geförderter Umgebungsluft aus dem Einatemzweig (3) wird durch das Auslaßgebläse (6) über das Auslaßfilter (9) in die Umgebung zurückgeblasen. Nach einer vorgegebenen Betriebszeit der Gebläse (5, 6) in Richtung der ausgezogenen Strömungspfeile (11) wird der Drehsinn der Gebläse (5, 6) umgekehrt, so daß nunmehr die Durchströmung der Filter (8, 9) und der Atemgasführung (2, 3, 4) der Richtung der gestrichelten Strömungspfeile (11) folgt (Fig. 1). Dabei wird der vorherige Ausatemzweig (4) zum neuen Einatemzweig, da nunmehr durch das Filter (9) die Umgebungsluft angesaugt und der Überschuß sowie die Ausatemluft durch das Filter (8) ausgeblasen wird. Während des Ausblasens aus Filter (8) werden die im Filtermaterial adsorbierten Schadstoffe zumindest teilweise, wenn nicht sogar vollständig desorbiert und aus dem Filter (8) in die Umgebung ausgetrieben. Dabei wird das Filter (8) in seiner Filterleistung regeneriert, so daß nach nochmaliger Umschaltung der Gebläse die ursprüngliche Durchströmungsrichtung (ausgezogene Pfeile (11)) wiederhergestellt wird. Jetzt kann das regenerierte Filter (8) wiederum als Einlaßfilter dienen, wobei das vorher beladene Filter (9) als Auslaßfilter regeneriert wird.

Dieselbe Regeneration der einzelnen Filter durch wechselweisen Betrieb als Einlaßfilter (8) und Auslaßfilter (9) wird durch die Schaltungsanordnung gemäß Figur 2 erzielt. Das für den Einatemzweig (3) und Ausatemzweig (4) gleicherweise geltende Gebläse (12) fördert die Luft kontinuierlich von dem Einlaßanschluß (13) in den Auslaßanschluß (14). In der dargestellten Grundstellung des Umschaltventils (15) werden in derselben Durchströmungsrichtung der Einatemzweig (3) und der Ausatemzweig (4) durchströmt. Erst bei Umschaltung des Ventils (15) von seiner Grundstellung in die Schaltstellung wird bei gleichbleibender Umdrehungs- und Förderrichtung des Gebläses (12) die Durchströmung von Einatemzweig (3) und Ausatemzweig (4) durch die sich überkreuzenden Schaltwege des Ventils (15) vertauscht.

## Ansprüche

1. Atemschutzgerät mit Gebläseunterstützung für die Durchströmung von mindestens zwei Filtern in der Atemgasführung, dadurch gekennzeichnet, daß jeweils eines (8) der Filter zeitweise in Einatemrichtung und das andere (9) zeitweise in Ausatemrichtung durchströmbar, und ihre Durchströmungsrichtung periodisch umkehrbar ist.

2. Atemschutzgerät nach Anspruch 1, dadurch gekennzeichnet, daß zur Umkehr der Durchströmungsrichtung der Drehsinn des an die jeweiligen Filter (8, 9) angeschlossenen Einzelgebläses (5, 6) umkehrbar ist.

3. Atemschutzgerät nach Anspruch 1, dadurch gekennzeichnet, daß zur Umkehr der Durchströmungsrichtung ein Umlenkventil (15) im Atemkreis (2, 3, 4) vorgesehen ist, welches an ein für den Atemkreis (2, 3, 4) gemeinsames Umwälzgebläse (12) angeschlossen ist.

4. Atemschutzgerät nach einem der Ansprüche 1 bis 3, dadurch gekennzeichnet, daß zwischen den Filtern (8, 9) und dem Atemanschluß (1) jeweils eine Trocknungspatrone (7) geschaltet ist.

Fig.1

Fig. 2